(19) **Europäisches Patentamt European Patent Office Office européen des brevets**

(11) **EP 2 440 049 B1**

(12) **EUROPEAN PATENT SPECIFICATION**

(45) Date of publication and mention
of the grant of the patent:
**13.12.2017 Bulletin 2017/50**

(51) Int Cl.:
*A61K 31/415* (2006.01)     *A01N 43/56* (2006.01)
*A61P 3/00* (2006.01)

(21) Application number: **10786733.5**

(22) Date of filing: **09.06.2010**

(86) International application number:
**PCT/US2010/037879**

(87) International publication number:
**WO 2010/144518 (16.12.2010 Gazette 2010/50)**

(54) **GENOTYPE SPECIFIC METHODS FOR TREATING HUMAN SUBJECTS USING 4-METHYLPYRAZOLE**

GENOTYPSPEZIFISCHE VERFAHREN ZUR BEHANDLUNG VON PERSONEN MIT 4-METHYLPYRAZOL

PROCÉDÉS SPÉCIFIQUES AU GÉNOTYPE PERMETTANT DE TRAITER LES SUJETS HUMAINS À L'AIDE DU 4-MÉTHYLPYRAZOLE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **10.06.2009 US 185884 P**

(43) Date of publication of application:
**18.04.2012 Bulletin 2012/16**

(73) Proprietor: **Horizon Orphan LLC
Lake Forest, IL 60045 (US)**

(72) Inventors:
• **DALEY, Thomas, E.
San Mateo, CA 94403 (US)**
• **SQUIERS, Elizabeth, C.
Half Moon Bay, CA 94019 (US)**
• **YU, Kin-Hung, Peony
Hillsborough, CA 94010 (US)**

(74) Representative: **Marshall, Cameron John
Carpmaels & Ransford LLP
One Southampton Row
London WC1B 5HA (GB)**

(56) References cited:
**US-A1- 2008 021 083     US-A1- 2008 021 083
US-A1- 2009 117 079**

• YIN, S. ET AL.: "Genetic polymorphism and activities of human lung alcohol and aldehyde dehydrogenases: implications for ethanol metabolism and cytotoxicity", BIOCHEMICAL GENETICS, vol. 30, no. 3-4, 1992, pages 203-215, XP009164120,
• GOEDDE, H.W. ET AL.: "Distribution od ADH2 and ALDH2 genotypes in different populations", HUM GENET, vol. 88, 1992, pages 344-346, XP002686012,

Remarks:
The file contains technical information submitted after the application was filed and not included in this specification

**Description**

## 1. <u>CROSS REFERENCE</u>

This application claims the benefit of U.S. Patent Application No. 61/185,884, filed June 10, 2009.

## 2. <u>TECHNICAL FIELD</u>

Disclosed herein is 4-methylpyrazole (4-MP), or physiologically acceptable salts thereof, for use in pharmaceutical methods in subjects of genetic subpopulations expressing specific polymorphisms of the alcohol dehydrogenase and aldehyde dehydrogenase genes. The present invention provides 4-MP, or physiologically acceptable salts thereof for use in methods to prevent or ameliorate ethanol intolerance, reduce or ameliorate symptoms associated with acetaldehyde accumulation accompanying ethanol consumption, or reduce the risk of diseases or disorders caused by consumption of ethanol, comprising orally administering 4-MP, or physiologically acceptable salts thereof, to subjects of these subpopulations, in a dosage of about 0.5 milligram to about 2 milligrams 4-MP or the equivalent mass of 4-MP in a physiologically acceptable salt form, per kilogram of the subject's body mass; and wherein the subject has the following genotype: i) ADH2*1/ADH2*1 and ALDH2*1/ALDH2*2; ii) ADH2*1/ADH2*2 and ALDH2*1/ALDH2*2; or iii) ADH2*1/ADH2*2 andALDH2*1/ALDH2*1.

## <u>BACKGROUND OF THE INVENTION</u>

Ethanol is consumed for a variety of social, recreational, and medicinal purposes in humans. Excessive ethanol consumption causes injury to a wide variety of tissues including liver, brain, skeletal, and cardiac muscle and is responsible for considerable public health morbidity and mortality.

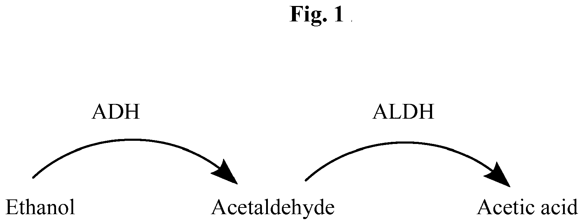

Many of these effects of ethanol are mediated by acetaldehyde, which is produced during ethanol metabolism in a two-step pathway in which ethanol is oxidized by alcohol dehydrogenase (ADH) to acetaldehyde, which is in turn quickly metabolized into acetic acid by aldehyde dehydrogenase (ALDH), a mitochondrial liver enzyme (*see* Figure 1).

The ADH and ALDH genes display polymorphisms that modulate individual differences in alcohol-oxidizing capability (Bosron et al., Hepatology 1986, 6, 502-510). East Asian populations carry a variant allele of alcohol dehydrogenase subtype 2 (ADH2*2) that encodes an ADH enzyme with a R47H amino acid substitution (Matsuo et al., Carcinogenesis 2006, 27(5), 1018-1023; Tamakoshi et al., Alcohol 2003, 38, 407-410). The H47 ADH enzyme is "superactive," exhibiting a $V_{max}$ about 40 times higher than the less active R47 ADH enzyme encoded by the "typical" allele (ADH2*1) (Bosron et al., Hepatology 1986, 6, 502-510; Yoshida et al., Prog. Nucleic Acid Res. Mol. Biol. 1991, 40, 255-287). The ADH2*2 allele is associated with the accumulation of acetaldehyde (Crabb et al., Proc. Nutr. Soc. 2004, 63(1), 49-63).

Also prevalent in East Asian populations is a variant allele of aldehyde dehydrogenase subtype 2 (ALDH2*2) that encodes for an ALDH enzyme with an E487K amino acid substitution (Chen et al., Am. J. Hum. Genet. 1999, 65(3), 795-807). The K487 ALDH enzyme exhibits reduced activity that results in a 40%-90% reduction in the rate of acetaldehyde removal when compared to the more active E487 ALDH2 enzyme encoded by the "typical" allele (ALDH2*1), such that persons who express the variant allele display reduced or absent ALDH2 activity.

Acetaldehyde is linked to acute symptoms such as facial flushing, tachycardia, shortness of breath, dizziness, nausea, vomiting and headache, as well as to increased long-term health risks for cancers of the upper digestive tract, breast cancer, liver disease, Alzheimer's disease, hypertension and myocardial infarction (*see* Visapää et al., Gut 2004, 53, 871-876; Yokoyama et al., Jpn. J. Clin. Oncol. 2003, 33(3), 111-121; Ohsawa et al., J. Hum. Genet. 2003, 48, 404-409). People who express the ALDH2*2 allele having reduced or absent ALDH2 activity exhibit alcohol-related sensitivity, for example, facial flushing, tachycardia, etc., when drinking small portions of ethanol (Goedde et al., Hum. Genet. 1992, 88, 344-346; Xiao et al., J. Clin. Invest. 1995, 96, 2180-2186). Therefore, reducing acetaldehyde levels in people with reduced or absent ALDH2 activity may be helpful in reducing the acute systems and long-term health risks experienced by these people when they consume ethanol.

US2008/021083 describes methods, articles of manufacture and compositions that are useful for the prevention or amelioration of a symptom of acetaldehyde accumulation or ethanol intolerance in a subject with reduced or absent reduced or absent aldehyde dehydrogenase subtype 2 (ALDH2) activity.

Yin, S. et al. Biochemical Genetics 1992, 30 (3-4), 344-346 describes genetic polymorphism and activities of human lung alcohol and aldehyde dehydrogenases: implications for ethanol metabolism and cytotoxicity.

Administration of 4-methylpyrazole (also known as fomepizole or 4-MP) has been shown to ameliorate symptoms of elevated acetaldehyde levels. *See, e.g.,* WO 2005/085392 and Japanese Unexamined Patent Application Publication No. S57-106620. However, it is becoming increasingly evident that 4-MP treatment is not suitable for all human genetic subpopulations. Additional information regarding individualized genetic factors that contribute to effective treatments with 4-MP is sought.

## 4. SUMMARY OF THE INVENTION

In one aspect of the disclosure, methods are provided for preventing, reducing or ameliorating a symptom of ethanol intolerance, or acetaldehyde accumulation, in a subject with reduced or absent aldehyde dehydrogenase subtype 2 (ALDH2) activity. In certain embodiments, the
subject is also homozygous for alcohol dehydrogenase subtype 2*1 (ADH2*1/ADH2*1) or is heterozygous for alcohol dehydrogenase subtypes 2*1 and 2*2 (ADH2*1/ADH2*2).

In certain aspects of the disclosure, methods are provided for preventing or ameliorating a symptom of ethanol intolerance in a subject with reduced or absent aldehyde dehydrogenase subtype 2 (ALDH2) activity comprising administering 4-MP, or a physiologically acceptable salt of 4-MP, to the subject. In certain embodiments, the subject is also homozygous for alcohol dehydrogenase subtype 2*1 (ADH2*1/ADH2*1) or is heterozygous for alcohol dehydrogenase subtypes 2*1 and 2*2 (ADH2*1/ADH2*2).

In certain aspects of the disclosure, methods are provided for preventing, reducing or ameliorating a symptom associated with acetaldehyde accumulation in a subject with reduced or absent aldehyde dehydrogenase subtype 2 (ALDH2) activity comprising administering 4-MP, or a physiologically acceptable salt of 4-MP, to the subject. In certain embodiments, the subject is also homozygous for alcohol dehydrogenase subtype 2*1 (ADH2*1/ADH2*1) or is heterozygous for alcohol dehydrogenase subtypes 2*1 and 2*2 (ADH2*1/ADH2*2). In certain embodiments, the acetaldehyde accumulation accompanies ethanol consumption.

A symptom of ethanol intolerance, or acetaldehyde accumulation, in a subject can be, for example, selected from the group consisting of flushing, elevated heart rate, palpitations, hypotension, nausea, dizziness, headache, vomiting, diarrhea, upset stomach, ataxia, and confused consciousness.

In another aspect of the disclosure, methods are provided for reducing a likelihood or risk in a subject with reduced or absent aldehyde dehydrogenase subtype 2 (ALDH2) for a disease or disorder caused by consumption of ethanol. In certain embodiments, the subject is also homozygous for alcohol dehydrogenase subtype 2*1 (ADH2*1/ADH2*1) or heterozygous for alcohol dehydrogenase subtypes 2*1 and 2*2 (ADH2*1/ADH2*2). Conditions or diseases associated with the consumption of ethanol can include, for example and without limitation, hangover, alcoholic gastritis, alcohol-induced liver damage, upper aerodigestive tract cancers, digestive tract cancers, breast cancer, late-onset Alzheimer's disease, hypertension, myocardial infarction, Parkinson's disease, amyotropic lateral sclerosis, and cerebral ischemia. In preferred embodiments, diseases associated with the consumption of ethanol include upper aerodigestive tract cancers, digestive tract cancers, breast cancer, late-onset Alzheimer's disease, hypertension, myocardial infarction, Parkinson's disease, amyotropic lateral sclerosis, and cerebral ischemia.

The invention also provides 4-MP or a physiologically acceptable salt thereof for use in methods for reducing a likelihood or risk in a subject for a disease or disorder caused by consumption of ethanol comprising administering to a subject in need thereof an amount of a 4-MP, or a physiologically acceptable salt of 4-MP in an amount between 0.5 and 2.0 mg/kg of 4-MP or the equivalent mass of 4-MP in a physiologically acceptable salt form, effective to increase catabolism of acetaldehyde in the subject, wherein the acetaldehyde is a product of ethanol consumption by the subject, thereby reducing a likelihood or risk in for a disease or disorder in the subject caused by exposure to acetaldehyde; and wherein the subject has the following genotype: i) ADH2*1/ADH2*1 and ALDH2*1/ALDH2*2; ii) ADH2*1/ADH2*2 and ALDH2*1/ALDH2*2; or iii) ADH2*1/ADH2*2 and ALDH2*1/ALDH2*1.

In certain embodiments of the disclosure, the methods provided comprise administering to the subject about 0.1 mg to about 5 mg 4-MP, or the equivalent mass of 4-MP in a physiologically acceptable salt form, per kilogram of the subject's body mass. In certain embodiments of the disclosure, the methods provided comprise administering to the subject about 0.1 mg to about 4 mg 4-MP, or the equivalent mass of 4-MP in a physiologically acceptable salt form, per kilogram of the subject's body mass. In certain embodiments of the disclosure, the methods provided comprise administering to the subject about 1 mg to about 4 mg 4-MP, or the equivalent mass of 4-MP in a physiologically acceptable salt form, per kilogram of the subject's body mass.

In the present invention, the compounds are for use in methods comprising administering to the subject 0.5 mg to 2 mg of 4-MP, or the equivalent mass of 4-MP in a physiologically acceptable salt form, per kilogram of the subject's body mass.

In certain embodiments, the compounds are for use in methods comprising administering to the subject 0.5 mg, about 1 mg, about 1.5 mg, or 2 mgof 4-MP, or the equivalent mass of 4-MP in a physiologically acceptable salt form, per kilogram of the subject's body mass.

In certain embodiments, 4-MP is orally administered.

In certain embodiments, 4-MP is orally administered before the subject consumes ethanol.

In certain embodiments, 4-MP is orally administered about two hours to about fifteen minutes before the subject consumes ethanol.

In other embodiments, 4-MP is orally administered concurrently with the subject's consumption of ethanol or after the subject has consumed ethanol.

In certain embodiments, the percent reduction in the subject's ethanol elimination rate is no more than about 10% in comparison to the ethanol elimination rate of a subject not administered 4-MP.

In certain embodiments, the compounds are for use in methods comprising administering an effective amount of 4-MP that reduces acetaldehyde accumulation by about 25% to about 60% as compared to a subject not administered 4-MP.

In certain embodiments, the compounds are for use in methods comprising administering an amount of 4-MP or a physiological acceptable salt of 4-MP effective to reduce or inhibit ethanol-oxidizing activity of alcohol dehydrogenase in the subject.

In certain embodiments, an effective amount of a hydrochloride salt of 4-MP is administered.

In certain embodiments, the ALDH2 and ADH2 genotypes of the subject are determined prior to administration of 4-MP.

In another aspect, compositions are provided which comprise 4-MP, or a physiologically acceptable salt thereof, and a physiologically acceptable excipient, suitable for oral administration to a subject.

In certain embodiments of the disclosure, the compositions may be for use preventing, reducing or ameliorating a symptom of ethanol intolerance, or a symptom associated with acetaldehyde accumulation accompanying ethanol consumption, in a subject with reduced or absent ALDH2 activity. In certain embodiments of the disclosure, the compositions may be used for reducing a risk for a disease or disorder caused by consumption of ethanol in a subject with reduced or absent ALDH2 activity.

In a further aspect of the disclosure, uses of 4-MP, or a pharmaceutically acceptable salt thereof, optionally in the manufacture of a medicament, are provided for preventing, reducing or ameliorating a symptom of ethanol intolerance, or a symptom associated with acetaldehyde accumulation accompanying ethanol consumption, in a subject with reduced or absent ALDH2 activity.

In a still further aspect of the disclosure, uses of 4-MP, or a pharmaceutically acceptable salt thereof, optionally in the manufacture of a medicament, are provided for reducing a risk for a disease or disorder caused by consumption of ethanol in a subject with reduced or absent ALDH2 activity.

## 5. DESCRIPTION OF FIGURES

**Figure 1.** Metabolism of ethanol.

**Figure 2.** Graph of mean serum acetaldehyde concentration measured in subjects with reduced ALDH2 activity, who are also ADH2*1/ADH2*1 homozygous or ADH2*1/ADH2*2 heterozygous, following dosage with 1 mg/kg 4-MP (filled circles); comparison with placebo (open circles).

**Figure 3.** Graph of mean serum acetaldehyde concentration measured in subjects with reduced or absent ALDH2 activity, who are also ADH2*2/ADH2*2 homozygous, following dosage with 1 mg/kg 4-MP (filled circles); comparison with placebo (open circles).

**Figure 4.** Graph of mean serum acetaldehyde concentration across all subjects with reduced or absent ALDH2 activity, following dosage with 4-MP (filled circles); comparison with placebo (open circles).

## 6. TERMINOLOGY

Generally, the nomenclature used herein and the laboratory procedures in medicinal chemistry, biochemistry, and pharmacology described herein are those well known and commonly employed in the art. Unless defined otherwise, all technical and scientific terms used herein generally have the same meaning as commonly understood by one of ordinary skill in the art to which this disclosure belongs. In the event that there is a plurality of definitions for a term used herein, those in this section prevail unless stated otherwise.

As used herein, "about" indicates a range of +/-10%. For example, "about 4 mg 4-MP" means a range of from 3.6 mg to 4.4 mg 4-MP.

The term "subject" refers to animals such as mammals, including, but not limited to, primates (*e.g.*, humans), monkeys, cattle, sheep, goats, horses, dogs, cats, rabbits, pigs, deer, bear, rats, mice and the like. In preferred embodiments, the subject is a human.

The terms "treat," "treating" or "treatment," as used herein, refers to a method of alleviating or abrogating a disorder and/or its attendant symptoms.

The terms "prevent," "preventing" or "prevention," in certain embodiments, refer to compounds for use in a method of barring a subject from acquiring a disorder and/or its attendant symptoms. In certain embodiments, the terms "prevent," "preventing," or "prevention," refer to compounds for use in a method of reducing the likelihood or risk of a subject acquiring a disorder and/or its attendant symptoms.

The term "physiologically acceptable salt," as used herein, refers to the relatively nontoxic, inorganic and organic acid addition salts of compounds of the invention.

The phrase "symptom of acetaldehyde accumulation accompanying ethanol consumption," as used herein refers to any symptom experienced by subjects with reduced or absent aldehyde dehydrogenase subtype 2 (ALDH2) activity when consuming ethanol. Symptoms can include, but are not limited to, flushing, elevated heart rate, palpitations, hypotension, nausea, dizziness, headache, vomiting, diarrhea, upset stomach, ataxia, or confused consciousness.

The phrase "subject with reduced or absent aldehyde dehydrogenase subtype 2 (ALDH2) activity" refers to a subject that is a homozygous or heterozygous carrier of the variant ALDH2*2 allele of the ALDH2 gene as described in Goedde et al., Hum. Genet. 1992, 88, 344-346, and Xiao et al., J. Clin. Invest. 1995, 96, 2180-2186, or to a subject that expresses any variant ALDH2 enzyme that exhibits less activity than the normal ALDH2 enzyme as determined by the aldehyde dehydrogenase activity assay described in Xiao et al., J. Clin. Invest. 1995, 96, 2180-2186.

The phrase "the subject is homozygous for alcohol dehydrogenase subtype 2*1 (ADH2*1/ADH2*1) or heterozygous for alcohol dehydrogenase subtypes 2*1 and 2*2 (ADH2*1/ADH2*2)" refers to a subject that is a homozygous or heterozygous carrier of the "typical" ADH2*1 allele.

As used herein, "ethanol intolerance," refers to a condition in which a subject experiences a symptom of acetaldehyde accumulation accompanying ethanol consumption.

As used herein, "ethanol elimination rate" refers to the reduction in ethanol concentration in a subject's bloodstream over time after the subject has ingested ethanol. Typically, an ethanol elimination rate can be expressed in terms of millimole ethanol/kilogram subject body mass/hour. Techniques for blood sampling and analysis of ethanol levels in blood are well known to those of skill in the art. *See, e.g.,* Inoue et al.,

Alcoholism: Clinical and Experimental Research 1984, 8, 319-322. A "percent change in ethanol elimination rate," can be calculated as follows:

$$\text{Percent Change in EtOH Elimination} = \left( 1 - \frac{\text{Subject's EtOH Elimination Rate After Taking 4-MP}}{\text{Subject's EtOH Elimination Rate Prior to Taking MP-4}} \right) \times 100$$

where EtOH represents ethanol, and a number for a percent change in ethanol elimination that is less than 100 is a reduction in the percent change in EtOH elimination. Blood ethanol levels can also be calculated, for example, based on algorithms utilizing the amount of ethanol consumed by a subject, the subject's body mass, and time period since the consumption of ethanol, or, as another example, blood ethanol levels can be extrapolated from analysis of a subject's breath, and the like, as known to those of skill in the art.

As used herein, "acetaldehyde accumulation" refers to the production of acetaldehyde in a subject that has consumed ethanol. Techniques for blood sampling and analysis of acetaldehyde levels in blood are well known to those of skill in the art. *See, e.g.,* Inoue et al., Alcoholism: Clinical and Experimental Research 1984, 8, 319-322. Also *see, e.g.,* Stowell, Clin. Chim. Acta. 1979, 98, 201-5, McCarver-May et al., Journal of Analytical Toxicology 1997, 21, 134-141, and Nagy et al., Rapid Communications in Mass Spectrometry 2004, 18, 2473-2478. Maximal concentrations of acetaldehyde accumulation typically follow fifteen minutes to one hour following ethanol consumption in a subject with reduced or absent ALDH2 activity. Where a "percent change in acetaldehyde accumulation" is used herein, this will be understood to mean the change in the maximal concentrations of acetaldehyde in a subject with reduced or absent ALDH2 activity, that can be calculated as follows:

$$\text{Percent Change in Acetaldehyde Accumulation} = \left( 1 - \frac{\text{Max. Acetaldehyde Conc. After Taking 4-MP}}{\text{Max. Acetaldehyde Conc. Prior to Taking MP-4}} \right) \times 100$$

where a number for a percent change in acetaldehyde accumulation that is less than 100 is a reduction in the percent change in acetaldehyde accumulation. Blood acetaldehyde concentrations can also be extrapolated from analysis of a subject's breath, or from measurable physiological changes in other parameters, such as heart rate or flushing, and the like, as known to those of skill in the art.

As defined herein, where the mass of 4-MP is specified, for example, "2 mg 4-MP," that amount refers to the equivalent mass of 4-MP in its free base form. Thus, for example, if 2 mg 4-MP in a given salt form is to be administered in a method disclosed herein, those of skill in the art can make the necessary conversion using the molecular masses of the salt form of 4-MP and of the free base form of 4-MP to determine the actual mass of that salt form of 4-MP necessary to obtain the equivalent mass of 2 mg 4-MP in its free base form. As another example, if 2 mg 4-MP in a free base form is to be administered in a method disclosed herein, then no conversion is necessary.

The term "effective amount" as used herein refers to the amount of 4-MP, or physiologically acceptable salt thereof, that is sufficient to produce a desirable or beneficial effect when contacted, for example, to an alcohol dehydrogenase enzyme, or, as another example, when administered to a subject. In certain embodiments, the "effective amount" is, for example, the amount to prevent, reduce or ameliorate a symptom associated with acetaldehyde accumulation in a subject accompanying ethanol consumption, or to reduce the likelihood or risk in a subject for a disease or disorder caused by consumption of ethanol.

The term "symptom" as used herein is interchangeable with "sign." Therefore, as used herein "symptom" refers to a physical condition which indicates a particular illness or disorder (*e.g.,* Longman Dictionary of Contemporary English 1995. Third Edition) detectable by the subject suffering from a particular disease or disorder or detectable by a person other than the subject without verbal information from said subject.

The term "AUC" as used herein, refers to the area under the curve of a plot of serum acetaldehyde concentration versus time following administration of 4-MP to a subject. In certain embodiments, the AUC is bounded by 0 to 8 hours ($AUC_{0-8H}$).

The term "AUC mean" as used herein, refers to the area under the curve of a plot of mean serum acetaldehyde concentration versus time following administration of 4-MP to subjects of a sample population. In certain embodiments, the AUC mean is bounded by 0 to 8 hours ($AUC_{0-8H}$ mean).

The term "$C_{max}$" as used herein, refers to the maximum serum acetaldehyde concentration following administration of 4-MP to a subject.

The term "$C_{max}$ mean" as used herein, refers to the mean maximum serum acetaldehyde concentration following administration of 4-MP to subjects of a sample population.

The term "P-value" as used herein, refers to the probability of obtaining a certain result. The lower the P-value, the less likely, and therefore, the more statistically significant the result. In certain embodiments, a P-value of 0.05 corresponds to a 5% chance of obtaining a certain result.

The term "SE" as used herein, refers to the standard error of the mean of a sample population. In certain embodiments, SE may refer to an estimate of the standard deviation calculated from sample data measured in a sample population.

## 7. DETAILED DESCRIPTION OF THE INVENTION

The present invention provides compounds for use in methods useful for reducing or ameliorating the severity of, or preventing, an adverse physiological symptom associated with acetaldehyde accumulation accompanying ethanol consumption in a subject, wherein the subject is a member of genetic subpopulations expressing specific polymorphisms of the alcohol dehydrogenase and aldehyde dehydrogenase genes, as further defined herein.

As explained below, the compounds for use in methods provided comprise the administration of 4-MP or a physiologically acceptable salt of 4-MP. Without intending to be bound by any particular theory or mechanism, it is believed that 4-MP acts to inhibit alcohol dehydrogenase (ADH) to reduce the accumulation of acetaldehyde production that results from the consumption of ethanol. Without intending to be bound by any particular theory or mechanism, it is also believed that certain genetic subpopulations of alcohol intolerant human subjects identified below will be more likely to benefit by the methods described herein, whereas other genetic subpopulations of alcohol intolerant human subjects identified below will be less likely to benefit by the methods described herein.

### 7.2. Methods to Prevent or Ameliorate a Symptom of Ethanol Intolerance or of Acetaldehyde Accumulation in Genetic Subpopulations of Human Subjects Expressing Specific Polymorphisms of the ALDH2 and ADH2 Genes

In one aspect of the disclosure, methods are provided for preventing, reducing or ameliorating a symptom of ethanol intolerance, or acetaldehyde accumulation, in a subject with reduced or absent aldehyde dehydrogenase subtype 2 (ALDH2) activity. In certain embodiments, the subject is also homozygous for alcohol dehydrogenase subtype 2*1 (ADH2*1/ADH2*1) or heterozygous for alcohol dehydrogenase subtypes 2*1 and 2*2 (ADH2*1/ADH2*2).

In certain embodiments, the compounds for use in methods comprise administering 4-methylpyrazole (4-MP) to the subject.

4-Methylpyrazole (4-MP, also known as fomepizole) is commercially available from chemical suppliers, including, for example, Sigma Aldrich (St. Louis, MO), and can also be synthesized easily in commercially viable quantities of pharmaceutical grade.

In certain embodiments, the ALDH2 and ADH2 genotypes of the subject are determined prior to administration of 4-MP.

In the present invention, the compound for use in the methods comprise administering 0.5 mg to 2 mg 4-MP per kilogram of a subject's body mass, to the subject.

In certain embodiments, the compound for use in the methods is the free base of 4-MP. In other embodiments, a physiologically acceptable salt of 4-MP can be used in the methods. In certain embodiments, a 4-MP hydrochloride salt can be used in the methods described herein.

4-MP can be administered alone or in combination with other substances or active agents. In certain embodiments, a composition comprising 4-MP and other ingredients, as described below, is administered.

4-MP can be administered according to any technique known to those of skill in the art. In certain embodiments, 4-MP can be delivered transdermally. In preferable embodiments, the subject can self-administer 4-MP to himself or herself. In preferable embodiments, 4-MP can be administered orally. When orally administered, 4-MP can be in a solid form, for example, as in a powder, tablet, capsule and the like, or in a liquid form.

In certain embodiments, the amount of 4-MP administered can be between 0.5 mg/kg to 2 mg/kg. In certain embodiments of the disclosure, the amount of 4-MP administered can be between about 0.1 mg/kg to about 4 mg/kg. In certain embodiments of the disclosure, the amount of 4-MP administered can be between about 1 mg/kg to about 5 mg/kg. In certain embodiments of the disclosure, the amount of 4-MP administered can be between about 1 mg/kg to about 4 mg/kg. As will be understood by those of skill in the art, the amounts of 4-MP to be administered, as described herein, are based on the body mass of the subject, expressed in kilograms. In some embodiments of the disclosure, about 0.1 mg/kg, about 0.5 mg/kg, about 1.5 mg/kg, about 2 mg/kg, about 2.5 mg/kg, about 3 mg/kg, about 3.5 mg/kg, about 4 mg/kg, about 4.5 mg/kg, or about 5 mg/kg of 4-MP are administered to the subject with reduced or absent aldehyde ALDH2 activity. In certain embodiments, the amount of 4-MP administered can be in the range between 0.5 mg/kg to 2 mg/kg.

In certain embodiments, the amount of 4-MP, or physiologically acceptable salt thereof, administered can be effective to reduce or inhibit the ethanol-oxidizing activity of alcohol dehydrogenase in the subject.

In certain embodiments, 4-MP can be administered before the subject has consumed ethanol. In certain embodiments, 4-MP can be administered about one minute, about fifteen minutes, or about one hour before the subject consumes ethanol. In certain embodiments, 4-MP can be orally administered about two hours to about fifteen minutes before the subject consumes ethanol.

In certain embodiments, 4-MP can be administered concurrent with the consumption of ethanol. In certain embodiments, 4-MP can be administered immediately before or after the consumption of ethanol. In certain embodiments, 4-MP can be administered to a subject after the subject has consumed ethanol.

It can be particularly advantageous to minimize the peak concentrations of acetaldehyde in the blood of subjects with reduced or absent ALDH2 activity, without a concomitant reduction in the rate of ethanol elimination, so as to avoid the consequence of having relatively lengthy periods of time during which the subject is under the influence of ethanol. With certain doses of 4-MP contemplated herein, that is, between 0.5 mg/kg to 2 mg/kg, the percent reduction in ethanol elimination rate can be negligibly or minimally impacted as discussed below.

In certain embodiments, compounds for use in methods are provided comprising the administration of 4-MP wherein a percent reduction in ethanol elimination ranges from about 0%, about 1%, about 2%, about 3%, about 4%, about 5% or about 6% to no more than about 10%. For example, if a subject not treated with 4-MP that has an ethanol elimination rate of 2.50 mmol/kg/hr, and has an ethanol elimination rate of 2.30 mmol/kg/hr when administered with 4-MP, than the percent reduction in ethanol elimination is 8%.

In certain embodiments, compounds for use in methods are provided that can have a percent reduction in the subject's ethanol elimination rate ranging from no reduction or 1-2% reduction in the ethanol elimination rate to less than about 7%, about 8%, about 9%, or about 10% reduction in the subject's rate of ethanol elimination. In certain embodiments, compounds for use in methods provided result in a reduction of ethanol elimination between about 5% to about 10%. In certain embodiments, the percent reduction in the subject's ethanol elimination rate is no more than about 10% in comparison to the ethanol elimination rate of the subject not treated with 4-MP.

With the doses of 4-MP contemplated in the instant methods, the percent reduction in peak blood acetaldehyde concentrations can be reduced in a subject with reduced ALDH2 activity.

In certain embodiments, the compounds for use in methods provided can reduce acetaldehyde accumulation by about 50% to about 60% in a subject with reduced or absent ALDH2 activity as compared to when 4-MP is not administered to the subject. In certain embodiments, the peak acetaldehyde accumulation can be effectively eliminated or reduced by about 95%, 90%, 85%, 80%, 75%, 70%, 65%, 60%, 55%, 50%, 45%, 40%, 35%, 30%, 25%, 20%, 15%, 10% or about 5%.

In certain embodiments, the compounds for use in methods provided prevent or ameliorate a symptom of ethanol intolerance, or acetaldehyde accumulation, in a subject selected from the group consisting of flushing, elevated heart rate, palpitations, hypotension, nausea, dizziness, headache, vomiting, diarrhea, upset stomach, ataxia, and confused consciousness.

**7.3. Methods to Reduce Likelihood or Risk in a Sul:Oct for a Disease or Disorder Caused by Consumption of Ethanol in Genetic Subpopulations of Human Sublects Expressing Specific Polymorphisms of the ALDH2 and ADH2 Genes**

In another aspect of the disclosure, methods are provided for reducing a likelihood or risk in a subject with reduced or absent aldehyde dehydrogenase subtype 2 (ALDH2) activity for a disease or disorder caused by consumption of ethanol. In certain embodiments, the subject is also homozygous for alcohol dehydrogenase subtype 2*1 (ADH2*1/ADH2*1) or heterozygous for alcohol dehydrogenase subtypes 2*1 and 2*2 (ADH2*1/ADH2*2).

In certain embodiments, the compound for use in the methods is the free base of 4-MP. In other embodiments, a physiologically acceptable salt of 4-MP can be used in the methods. In certain embodiments, a 4-MP hydrochloride salt can be used in the methods described herein.

In certain embodiments, the compound for use in methods comprise administering 0.5 mg to 2 mg 4-MP per kilogram of a subject's body mass, to the subject.

In certain embodiments, the ALDH2 and ADH2 genotypes of the subject are determined prior to administration of 4-MP.

In certain embodiments, the compounds for use in methods are effective to increase catabolism of acetaldehyde in the subject, thereby reducing a likelihood or risk in for a disease or disorder caused by exposure to acetaldehyde in the subject.

In certain embodiments, the acetaldehyde is a product of ethanol consumption by the subject.

In certain embodiments, the disease or disorder comprises upper aerodigestive tract cancers, digestive tract cancers or breast cancer. In an additional embodiment the upper aerodigestive tract cancer comprises esophageal, oropharynx, hypopharynx, larynx, head or neck cancer. In a further embodiment the digestive cancer comprises stomach or colon cancer.

In certain embodiments, the disease or disorder comprises late-onset Alzheimer's disease, hypertension, myocardial infarction, Parkinson's disease, amyotropic lateral sclerosis, and cerebral ischemia.

## 8. EXAMPLES

**8.1. Reducing Acetaldehyde Concentration in Genetic Subpopulations of Human Subjects Expressing Specific Polymorphisms of the ALDH2 and ADH2 Genes**

The example demonstrates that 4-MP works to reduce acetaldehyde accumulation accompanying ethanol consumption in genetic subpopulations of human subjects expressing specific polymorphisms of the ALDH2 and ADH2 genes.

### 8.1.1. Methods

A blinded, intra-subject controlled, single dose, dose escalation study of 4-MP for mitigation of acetaldehyde related toxicity in subjects with symptoms of inborn altered ethanol metabolism with concomitant ethanol exposure was undertaken.

Thirty-two (n = 32) healthy human subjects of Japanese descent, 21 to 50 years of age, known to have intolerance to ethanol due to a history of flushing, heart palpitations, and/or nausea following ethanol consumption, and exhibiting a positive result to a skin ethanol patch test, were selected for the study population.

Twenty-four subjects (n = 24) were randomly selected and orally administered 1 mg/kg, 3 mg/kg, or 5 mg/kg of 4-MP, followed 30 minutes later by an oral dose of 20% ethanol (5 mg/kg). The dosing protocol is shown in Table 1.

**Table 1:** Dosing protocol for 4-MP

| 4-MP Dose | 4-MP then ETOH | ETOH then 4-MP |
|---|---|---|
| 1 mg/kg | 12 subjects | 4 subjects |
| 3 mg/kg | 4 subjects | 4 subjects |
| 5 mg/kg | 8 subjects | |

Using the same protocol, wherein 4-MP was replaced with placebo, each of the twenty-four randomly selected subjects served as its own control.

The twenty-four randomly selected subjects were then subjected to blood sampling, and the sampled blood analyzed for acetaldehyde levels at select time points following ethanol consumption.

For acetaldehyde analysis, blood samples were prepared by dilution with an internal standard (acetaldehyde-d4), with derivatization of acetaldehyde with dinitrophenyl hydrazine in hydrochloric acid, liquid-liquid portioning and solvent exchange. The derivatization forms an acetaldehyde-dinitrophenyl hydrazine (DNPH) derivative which dramatically increases the boiling point of the molecule and thus its relative stability compared to the acetaldehyde itself (Nagy et al., Rapid Communications in Mass Spectrometry 2004, 18, 2473-2478). The derivatized analytes were separated using a Jones Chromatography (Grace-Vydac), 50 mm x 2.1 mm, 3 $\mu$m column held in an oven set at 28 °C. Two mobile phases were used in a gradient consisting of 30:70 acetonitrile:water versus 90:10 acetonitrile:water with 0.1% formic acid. The derivatized analyte was detected on a High Performance Liquid Chromatograph equipped with a Mass Spectrometer (HPLC/MS/MS). Quantitation was performed using a linear regression curve fit of impurities standards with 1/x weighting, prepared from dilutions of acetaldehyde in water. Samples and standards were injected at 2 $\mu$L.

All thirty-two subjects were then genotyped for ALDH2 and ADH2 at the Alcohol Research Center at Indiana University using (i) genomic DNA isolation and (ii) TaqMan assay for allelic discrimination.

i. <u>Genomic DNA isolation</u>: DNA was first isolated using the "HotSHOT" method for isolating PCR quality genomic DNA (Truett et al., Biotechniques 2000, 29(1), 52, 54). Briefly, blood blotted on filter paper (3 mm circle) was placed in the well of a 96-well plate and mixed with 75 $\mu$L 25mM NaOH/0.3mM EDTA solution and heated to 95 °C for 40 minutes, followed by neutralization with 75 $\mu$L 40 mM Tris-HCl (pH 5) solution. 1-5 microliters were used for each PCR.

ii. <u>TaqMan Assay for Allelic Discrimination</u>: The allelic discrimination assay used a multiplexed (more than one primer/probe pair per reaction), end-point (data is collected at the end of the PCR process) assay. Each assay mix contained two different TaqMan probes (Applied BioSystems, Foster City, CA), labeled with VIC or FAM fluorescent reporter dye, which bound preferentially to one of the alleles. The genotype of each sample was determined by the fluorescence levels of the reporter dyes and was clustered on a graph with other samples of the same genotype. Each reaction contained 5 $\mu$L of 2XTaqMan Universal PCR Mastermix, No AmpErase UNG, 3.75 $\mu$L of water, 0.25 $\mu$L of 40X Assay Mix, and 1 $\mu$L of DNA sample. Eight or eleven controls were included on each 96-well plate: two no template controls, two or three heterozygous samples, and two or three of each of the homozygous samples. Thermocycling was carried out in MJ Research PTC-200 thermocyclers. The PCR products were then analyzed in an ABI PRISM® 7300 Sequence Detection System (SDS) instrument. SDS Software 1.3.1 converted the raw data to pure dye components and ploted the results of the allelic discrimination on a scatter plot of Allele X versus Allele Y (*e.g.,* ADH2*1 versus ADH2*2); each genotype appeared on the graph as a cluster of points.

On the basis of the ALDH2 and ADH2 genotyping described above, subjects were found to fall into seven genotypic groups (Groups A, B, C, D, E, F and G):

<u>Group A</u>: heterozygous for aldehyde dehydrogenase subtypes 2*1 and 2*2 (ALDH2*1/ ALDH2*2), and homozygous for alcohol dehydrogenase subtype 2*1 (ADH2*1 /ADH2*1); there were two subjects (n = 2) in this group;

<u>Group B</u>: homozygous for aldehyde dehydrogenase subtype 2*2 (ALDH2*2/ ALDH2*2), and homozygous for alcohol dehydrogenase subtype 2*1 (ADH2*1/ADH2*1); there was one subject (n = 1) in this group;

<u>Group C</u>: homozygous for aldehyde dehydrogenase subtype 2*1 (ALDH2*1/ ALDH2*1), and heterozygous for alcohol dehydrogenase subtypes 2*1 and 2*2 (ADH2*1/ADH2*2); there were four subjects (n = 4) in this group;

<u>Group D</u>: heterozygous for aldehyde dehydrogenase subtypes 2*1 and 2*2 (ALDH2*1/ ALDH2*2), and heterozygous for alcohol dehydrogenase subtypes 2*1 and 2*2 (ADH2*1/ADH2*2); there were six subjects (n = 6) in this group;

<u>Group E</u>: homozygous for aldehyde dehydrogenase subtype 2*1 (ALDH2*1/ ALDH2*1), and homozygous for alcohol dehydrogenase subtype 2*2 (ADH2*2/ADH2*2); there were five subjects (n = 5) in this group;

<u>Group F</u>: heterozygous for aldehyde dehydrogenase subtypes 2*1 and 2*2 (ALDH2*1/ ALDH2*2), and homozygous for alcohol dehydrogenase subtype 2*2 (ADH2*2/ADH2*2); there were thirteen subjects (n = 13) in this group; and

<u>Group G</u>: homozygous for aldehyde dehydrogenase subtype 2*2 (ALDH2*2/ALDH2*2), and homozygous for alcohol dehydrogenase subtype 2*2 (ADH2*2/ADH2*2); there was one subject (n = 1) in this group.

Table 2 indicates the ADLH2 and ADH2 genotype of each of the thirty-two subjects.

Table 2: ADLH2 and ADH2 genotype of study subjects

|  | ADH2*1/ADH2*1 Normal ADH2 activity | ADH2*1/ADH2*2 Enhanced ADH2 activity | ADH2*2/ADH2*2 "Superactive"ADH2 |
|---|---|---|---|
| ALDH2*1/ALDH2*1 Normal ALDH2 activity |  | Group C (4 subjects) | Group E (5 subjects) |
| ALDH2*1/ALDH2*2 Reduced ALDH2 activity | Group A (2 subjects) | Group D (6 subjects) | Group F (13 subjects) |
| ALDH2*2/ALDH2*2 Absent ALDH2 activity | Group B (1 subject) |  | Group G (1 subject) |

Data pertaining to acetaldehyde concentrations measured from the blood samples of subjects from each of the seven groups described above were compared as a function of genotype.

**8.1.2 Results**

8.1.2.1. Groups A and D

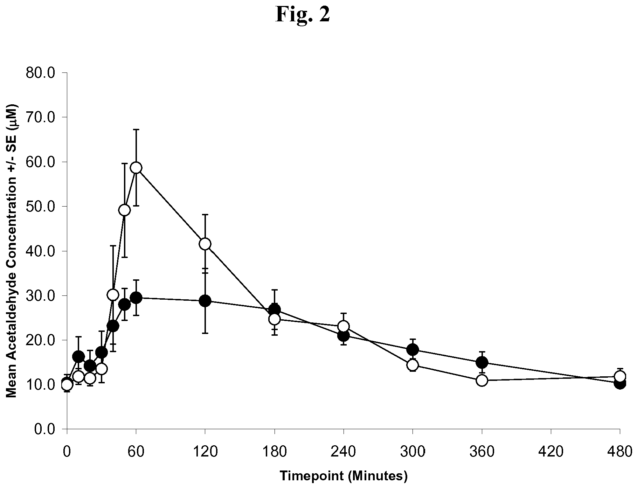

Figure 2 presents a graph of mean serum acetaldehyde concentration measured in six subjects (n = 6) with reduced ALDH2 activity, who are also ADH2*1/ADH2*1 homozygous or ADH2*1/ADH2*2 heterozygous (Groups A and D), following dosage with 1 mg/kg 4-MP, and comparison with placebo.

Table 3 presents the pharmacokinetic parameters of "$C_{max}$ mean" and "$AUC_{0-8H}$ mean" calculated from the data presented in Figure 2 using standard mathematical models known to those of skill in the art. P-values and standard errors were determined from statistical analysis of the data and are also presented in Table 3.

Table 3: $C_{max}$ mean and $AUC_{0-8H}$ mean calculated from the data presented in Figure 2

|  | 4-MP (n = 6) | Placebo (n = 6) | P-value |
|---|---|---|---|
| $C_{max}$ mean (µg/ml) (SE) | 41.3 (5.2) | 64.7 (8.4) | 0.029 |
| Acetaldehyde $AUC_{0-8H}$ mean(µg*hr/mL) (SE) | 162 (18.7) | 186 (14.8) | 0.071 |

Figure 2 demonstrates that the peak mean acetaldehyde concentration in the blood due to drinking ethanol can be reduced in subjects with reduced ALDH2 activity, who are also ADH2*1/ADH2*1 homozygous or ADH2*1/ADH2*2 heterozygous, following administration with 1 mg/kg 4-MP.

Table 3 demonstrates that the $C_{max}$ mean and $AUC_{0-8H}$ mean of acetaldehyde concentration in the blood due to drinking ethanol can be significantly reduced in subjects with reduced ALDH2 activity, who are also ADH2*1/ADH2*1 homozygous or ADH2*1/ADH2*2 heterozygous, following administration with 1 mg/kg 4-MP, relative to the $C_{max}$ mean and $AUC_{0-8H}$ mean in those subjects after drinking ethanol in the absence of 4-MP. For example, according to Table 3, $C_{max}$ mean is reduced from 64.7 $\pm$ 8.4 µg/mg to 41.3 $\pm$ 5.2 µg/mg, and $AUC_{0-8H}$ mean is reduced from 186 $\pm$ 14.8 µg*hr/mL to 162 $\pm$ 18.7 µg*hr/mL, relative to placebo. The reduction in $C_{max}$ mean represents a reduction of approximately 36%.

These results demonstrate that 1 mg/kg 4-MP is effective in reducing acetaldehyde accumulation accompanying ethanol consumption in certain genetic subpopulations of subjects with reduced ALDH2 activity, for example, those subjects who are also ADH2*1/ADH2*1 homozygous or ADH2*1/ADH2*2 heterozygous.

8.1.2.2. Groups F and G

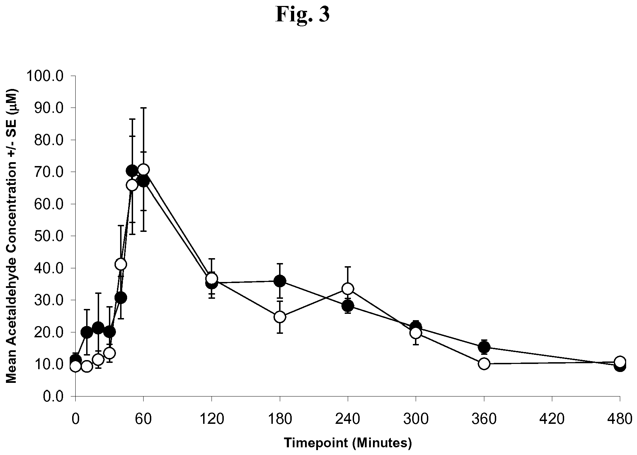

Figure 3 presents a graph of mean serum acetaldehyde concentration measured in six subjects (n = 6) with reduced or absent ALDH2 activity, who are also ADH2*2/ADH2*2 homozygous (Groups F and G), following dosage with 1 mg/kg 4-MP, and comparison with placebo.

Table 4 presents the pharmacokinetic parameters of "$C_{max}$ mean" and "$AUC_{0-8H}$ mean" calculated from the data presented in Figure 3 using standard mathematical models known to those of skill in the art. P-values and standard errors were determined from statistical analysis of the data and are also presented in Table 4.

**Table 4:** $C_{max}$ mean and $AUC_{0-8H}$ mean calculated from the data presented in Figure 3

|  | 4-MP (n = 6) | Placebo (n = 6) | P-value |
|---|---|---|---|
| $C_{max}$ mean (µg/ml)<br>(SE) | 89.2<br>(10.0) | 83.2<br>(8.4) | 0.351 |
| Acetaldehyde $AUC_{0-8H}$ mean(µg*hr/mL)<br>(SE) | 221<br>(11.6) | 206<br>(30.0) | 0.338 |

Figure 3 demonstrates that the peak mean acetaldehyde concentration in the blood due to drinking ethanol is not reduced in subjects with reduced or absent ALDH2 activity, who are also ADH2*2/ADH2*2 homozygous, following administration with 1 mg/kg 4-MP.

Table 4 demonstrates that the $C_{max}$ mean and $AUC_{0-8H}$ mean of acetaldehyde concentration in the blood due to drinking ethanol is not significantly reduced in subjects with reduced or absent ALDH2 activity, who are also ADH2*2/ADH2*2 homozygous, following administration with 1 mg/kg 4-MP, relative to the $C_{max}$ mean and $AUC_{0-8H}$ mean in those subjects after drinking ethanol in the absence of 4-MP.

These results indicate that certain genetic subpopulations of subjects with reduced or absent ALDH2 activity, for example, those subjects who are also ADH2*2/ADH2*2 homozygous, are less likely to be benefitted by administration of certain doses of 4-MP, for example, 1 mg/kg 4-MP. This result indicates the presence of a differential response to the administration of 4-MP in alcohol intolerant human subjects.

8.1.2.3. Groups A, B, D, F and G

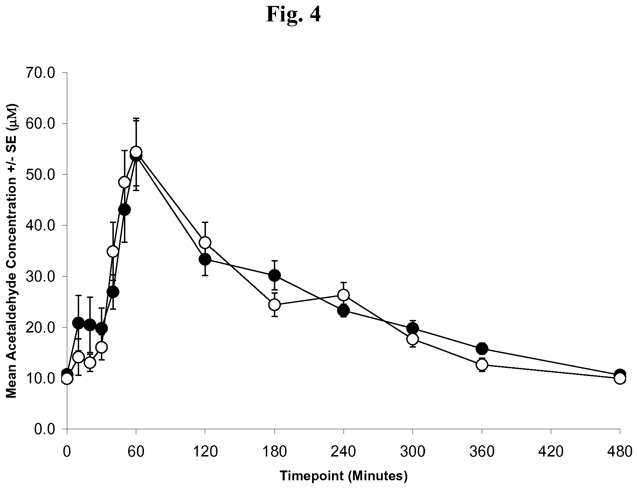

Figure 4 presents a graph of mean serum acetaldehyde concentration across twenty-three subjects (n = 23) with reduced or absent ALDH2 activity (Groups A, B, D, F and G), following dosage with 1 mg/kg, 3 mg/kg or 5 mg/kg 4-MP, and comparison with placebo.

Table 5 presents the pharmacokinetic parameters of "$C_{max}$ mean" and "$AUC_{0-8H}$ mean" calculated from the data presented in Figure 4 using standard mathematical models known to those of skill in the art. P-values and standard errors were determined from statistical analysis of the data and are also presented in Table 5.

**Table 5:** $C_{max}$ mean and $AUC_{0-8H}$ mean calculated from the data presented in Figure 4

|  | 4-MP (n = 23) | Placebo (n = 23) | P-value |
|---|---|---|---|
| $C_{max}$ mean (µg/ml)<br>(SE) | 64.2<br>(7.0) | 66.6<br>(6.3) | 0.367 |
| Acetaldehyde $AUC_{0-8H}$ mean (µg*hr/mL)<br>(SE) | 182<br>(10.5) | 188<br>(13.5) | 0.307 |

Figure 4 demonstrates that the peak mean acetaldehyde concentration in the blood due to drinking ethanol is not reduced across all subjects with reduced or absent ALDH2 activity, following administration with 1 mg/kg, 3 mg/kg or 5 mg/kg 4-MP.

Table 5 demonstrates that the $C_{max}$ mean and $AUC_{0-8H}$ mean of acetaldehyde concentration in the blood due to drinking ethanol is not significantly reduced in all subjects with reduced or absent ALDH2 activity, following administration with 1 mg/kg, 3 mg/kg or 5 mg/kg 4-MP, relative to the $C_{max}$ mean and $AUC_{0-8H}$ mean in those subjects after drinking ethanol in the absence of 4-MP.

These results indicate that certain genetic subpopulations of subjects with reduced or absent ALDH2 activity, for example, those subjects who are also ADH2*2/ADH2*2 homozygous, are less likely to be benefitted by administration of doses of 4-MP within the range of 1-5 mg/kg.

Taken together, the above studies indicate the presence of a differential response to the administration of 4-MP in alcohol intolerant human subjects: certain genetic subpopulations of alcohol intolerant human subjects, for example, subjects with reduced ALDH2 activity who are also ADH2*1/ADH2*1 homozygous or ADH2*1/ADH2*2 heterozygous, are more likely to be benefitted by the administration of 4-MP within the range of 1-5 mg/kg than other subpopulations of alcohol intolerant human subjects, for example, subjects with reduced ALDH2 activity who are also ADH2*2/ADH2*2 homozygous.

### 8.2. Exemplary Administration of 4-MP to a Human Subject

The example below describes exemplary administration of 4-MP to a human subject.

4-MP in its free base, liquid form, is mixed with orange juice to make a 0.5% (w/v) 4-MP solution. The 4-MP may be stored in a container with an associated dispensing cup with markings indicating various amounts of solution to be used for different body masses of people to whom the 4-MP will be administered. For a person with a body mass of about 75 kg with reduced or absent ALDH2 activity who will be drinking ethanol, about 60 milliliters of the 4-MP is poured into the dispensing cup and the person with reduced or absent ALDH2 can drink the 4-MP solution from the cup in the minutes or hours prior to drinking alcohol.

Although the foregoing invention has been described in some detail by way of illustration and example for purposes of clarity of understanding, it will be apparent to those skilled in the art that certain changes and modifications will be practiced. Therefore, the description and examples should not be construed as limiting the scope of the invention, which is delineated by the appended claims.

### Claims

1. 4-Methylpyrazole (4-MP) or a physiologically acceptable salt thereof for use in a method for prevention or amelioration of a symptom of ethanol intolerance or acetaldehyde accumulation accompanying ethanol consumption, or a method of reducing the risk of a disease caused by consumption of ethanol in a subject, wherein the method comprises oral administration of 4-MP or a physiologically acceptable salt thereof in a dosage of about 0.5 milligram to about 2 milligrams 4-MP or the equivalent mass of 4-MP in a physiologically acceptable salt form, per kilogram of the subject's body mass;
and wherein the subject has the following genotype:

   i) ADH2*1/ADH2*1 and ALDH2*1/ALDH2*2;
   ii) ADH2*1/ADH2*2 and ALDH2*1/ALDH2*2; or
   iii) ADH2*1/ADH2*2 and ALDH2*1/ALDH2*1.

2. The 4-MP or physiologically acceptable salt thereof for use of claim 1, wherein the symptom of ethanol intolerance is selected from the group consisting of flushing, elevated heart rate, palpitations, hypotension, nausea, dizziness, headache, vomiting, diarrhea, upset stomach, ataxia, and confused consciousness.

3. The 4-MP or physiologically acceptable salt thereof for use of claim 1, wherein the method comprises administration of 4-MP in a free base form or in a physiologically acceptable salt form.

4. The 4-MP or physiologically acceptable salt thereof for use of claim 1, wherein the method comprises administration of 4-MP or a physiologically acceptable salt thereof orally before the subject consumes ethanol, or concurrently with the subject's consumption of ethanol or after the subject has consumed ethanol.

5. The 4-MP or physiologically acceptable salt thereof for use of claim 4, wherein the method comprises administration of 4-MP or a physiologically acceptable salt thereof orally about one hour to about fifteen minutes before the subject consumes ethanol.

6. The 4-MP or physiologically acceptable salt thereof for use of claim 1, wherein the percent reduction in the subject's ethanol elimination rate is no more than about 10% in comparison to the ethanol elimination rate of a subject not administered 4-MP.

7. The 4-MP or physiologically acceptable salt thereof for use of claim 1, wherein the amount of 4-MP or physiologically acceptable salt thereof is effective to reduce or inhibit ethanol-oxidizing activity of alcohol dehydrogenase in the subject.

8. The 4-MP or physiologically acceptable salt thereof for use of claim 7, wherein the symptom of acetaldehyde accumulation is selected from the group consisting of flushing, elevated heart rate, palpitations, hypotension, nausea, dizziness, headache, vomiting, diarrhea, upset stomach, ataxia and confused consciousness.

9. Methylpyrazole (4-MP) or a physiologically acceptable salt thereof for use in a method for reduction of a risk in a subject for a disease or disorder caused by consumption of ethanol, wherein the method comprises administration of 4-MP or a physiologically acceptable salt of 4-MP in an amount between 0.5 and 2.0 mg/kg of 4-MP or the equivalent mass of 4-MP in a physiologically acceptable salt form, which is effective to increase catabolism of acetaldehyde in the subject, wherein the acetaldehyde is a product of ethanol consumption by the subject and wherein increasing catabolism of acetaldehyde reduces a risk for a disease or disorder in the subject caused by exposure to acetaldehyde;

and wherein the subject has the following genotype:

    i) ADH2*1/ADH2*1 and ALDH2*1/ALDH2*2;
    ii) ADH2*1/ADH2*2 and ALDH2*1/ALDH2*2; or
    iii) ADH2*1/ADH2*2 and ALDH2*1/ALDH2*1.

10. The 4-MP or physiologically acceptable salt thereof for use of either one of claims 1 and 9, wherein the subject is a human.

11. The 4-MP or physiologically acceptable salt thereof for use of claim 1, wherein the disease or disorder comprises upper aerodigestive tract cancers, digestive tract cancers or breast cancer, or wherein the disease or disorder comprises late-onset Alzheimers disease, hypertension, myocardial infarction, Parkinson's disease, amyotropic lateral sclerosis, and cerebral ischemia.

12. The 4-MP or physiologically acceptable salt thereof for use of claim 11, wherein the upper aerodigestive tract cancer comprises esophageal, oropharynx, hypopharynx, larynx, head or neck cancer or wherein the digestive cancer comprises stomach or colon cancer.

13. The 4-MP or physiologically acceptable salt thereof for use of either one of claims 1 and 9, wherein the method comprises administration of an effective amount of a hydrochloride salt of 4-MP.

14. The 4-MP or physiologically acceptable salt thereof for use of either one of claims 1 and 9, wherein the method comprises administration of about 1 milligram of 4-MP per kilogram of subject body mass.

**Patentansprüche**

1. 4-Methylpyrazol (4-MP) oder ein physiologisch unbedenkliches Salz davon zur Verwendung bei einem Verfahren zur Vorbeugung oder Linderung eines Symptoms der Ethanolintoleranz oder Acetaldehydanreicherung als Begleiterscheinung von Ethanolkonsum oder einem Verfahren zur Verringerung des Risikos einer durch Konsum von Ethanol verursachten Krankheit bei einem Individuum, wobei das Verfahren orale Verabreichung von 4-MP oder eines physiologisch unbedenklichen Salzes davon in einer Dosierung von etwa 0,5 Milligramm bis etwa 2 Milligramm 4-MP oder der äquivalenten Masse von 4-MP in einer physiologisch unbedenklichen Salzform pro Kilogramm Körpermasse des Individuums umfasst; und wobei das Individuum den folgenden Genotyp aufweist:

    i) ADH2*1/ADH2*1 und ALDH2*1/ALDH2*2;
    ii) ADH2*1/ADH2*2 und ALDH2*1/ALDH2*2; oder
    iii) ADH2*1/ADH2*2 und ALDH2*1/ALDH2*1.

2. 4-MP oder physiologisch unbedenkliches Salz davon zur Verwendung nach Anspruch 1, wobei das Symptom der Ethanolintoleranz aus der aus Hitzewallungen, erhöhter Herzfrequenz, Herzklopfen, Hypotonie, Übelkeit, Schwindel, Kopfschmerzen, Erbrechen, Diarrhö, Magenverstimmung, Ataxie und Bewusstseinsstörung bestehenden Gruppe ausgewählt ist.

3. 4-MP oder physiologisch unbedenkliches Salz davon zur Verwendung nach Anspruch 1, wobei das Verfahren eine Verabreichung von 4-MP in freier Basenform oder in physiologisch unbedenklicher Salzform umfasst.

4. 4-MP oder physiologisch unbedenkliches Salz davon zur Verwendung nach Anspruch 1, wobei das Verfahren eine orale Verabreichung von 4-MP oder eines physiologisch unbedenklichen Salzes davon vor dem Konsumieren von Ethanol durch das Individuum oder gleichzeitig mit dem Konsumieren von Ethanol durch das Individuum oder nach dem Konsumieren von Ethanol durch das Individuum umfasst.

5. 4-MP oder physiologisch unbedenkliches Salz davon zur Verwendung nach Anspruch 4, wobei das Verfahren eine orale Verabreichung von 4-MP oder eines physiologisch unbedenklichen Salzes davon etwa eine Stunde bis etwa fünfzehn Minuten vor dem Konsumieren von Ethanol durch das Individuum umfasst.

6. 4-MP oder physiologisch unbedenkliches Salz davon zur Verwendung nach Anspruch 1, wobei die prozentuale Verringerung der Ethanoleliminierungsrate des Individuums nicht mehr als etwa 10% im Vergleich mit der Ethanoleliminierungsrate eines Individuums, dem kein 4-MP verabreicht wurde, beträgt.

7. 4-MP oder physiologisch unbedenkliches Salz davon zur Verwendung nach Anspruch 1, wobei die Menge an 4-MP bzw. des physiologisch unbedenklichen Salzes davon wirksam ist, um die Ethanol oxidierende Aktivität von Alkohol-Dehydrogenase bei dem Individuum zu reduzieren oder zu hemmen.

8. 4-MP oder physiologisch unbedenkliches Salz davon zur Verwendung nach Anspruch 7, wobei das Symptom der Acetaldehydanreicherung aus der aus Hitzewallungen, erhöhter Herzfrequenz, Herzklopfen, Hypotonie, Übelkeit, Schwindel, Kopfschmerzen, Erbrechen, Diarrhö, Magenverstimmung, Ataxie und Bewusstseinsstörung bestehenden Gruppe ausgewählt ist.

9. Methylpyrazol (4-MP) oder ein physiologisch unbedenkliches Salz davon zur Verwendung bei einem Verfahren zur Verringerung eines Risikos einer durch Konsum von Ethanol verursachten Krankheit oder Störung bei einem Individuum, wobei das Verfahren Verabreichung von 4-MP oder eines physiologisch unbedenklichen Salzes von 4-MP in einer Menge zwischen 0,5 und 2,0 mg/kg von 4-MP oder der äquivalenten Masse von 4-MP in einer physiologisch unbedenklichen Salzform, die wirksam ist, um den Acetaldehyd-Katabolismus beim Individuum zu steigern, wobei es sich bei dem Acetaldehyd um ein Produkt des Ethanolkonsums durch das Individuum handelt und wobei eine Steigerung des Acetaldehyd-Katabolismus ein Risiko einer durch Inkontaktkommen mit Acetaldehyd verursachten Krankheit oder Störung bei dem Individuum verringert, umfasst; und wobei das Individuum den folgenden Genotyp aufweist:

i) ADH2*1/ADH2*1 und ALDH2*1/ALDH2*2;
ii) ADH2*1/ADH2*2 und ALDH2*1/ALDH2*2; oder
iii) ADH2*1/ADH2*2 und ALDH2*1/ALDH2*1.

10. 4-MP oder physiologisch unbedenkliches Salz davon zur Verwendung nach einem der Ansprüche 1 und 9, wobei es sich bei dem Individuum um einen Menschen handelt.

11. 4-MP oder physiologisch unbedenkliches Salz davon zur Verwendung nach Anspruch 1, wobei die Krankheit oder Störung Krebserkrankungen der oberen Luft- und Verdauungswege, Krebserkrankungen des Verdauungstrakts oder Brustkrebs umfasst oder wobei die Krankheit oder Störung spät einsetzenden Morbus Alzheimer, Bluthochdruck, Myokardinfarkt, Morbus Parkinson, amyotrophe Lateralsklerose und zerebrale Ischämie umfasst.

12. 4-MP oder physiologisch unbedenkliches Salz davon zur Verwendung nach Anspruch 11, wobei die Krebserkrankung der oberen Luft- und Verdauungswege Ösophagus-, Oropharynx-, Hypopharynx-, Larynxkarzinom, Krebs des Kopf- und Halsbereichs umfasst oder wobei die Krebserkrankung des Verdauungssystems Magen- oder Darmkrebs umfasst.

13. 4-MP oder physiologisch unbedenkliches Salz davon zur Verwendung nach einem der Ansprüche 1 und 9, wobei das Verfahren Verabreichung einer wirksamen Menge eines Hydrochloridsalzes von 4-MP umfasst.

14. 4-MP oder physiologisch unbedenkliches Salz davon zur Verwendung nach einem der Ansprüche 1 und 9, wobei das Verfahren Verabreichung von etwa 1 Milligramm 4-MP pro Kilogramm Körpermasse des Individuums umfasst.

## Revendications

1. 4-Méthylpyrazole (4-MP) ou sel physiologiquement acceptable de celui-ci pour utilisation dans une méthode de prévention ou de soulagement d'un symptôme d'intolérance à l'éthanol ou d'accumulation d'acétaldéhyde accompagnant la consommation d'éthanol, ou une méthode de réduction du risque d'une maladie provoquée par la consommation d'éthanol chez un sujet, où la méthode comprend l'administration orale de 4-MP ou d'un sel physiologiquement acceptable de celui-ci à une dose d'environ 0,5 milligramme à environ 2 milligrammes de 4-MP ou la masse équivalente de 4-MP sous une forme saline physiologiquement acceptable, par kilogramme de la masse

14

corporelle du sujet ;
et où le sujet présente le génotype suivant :

    i) ADH2*1/ADH2*1 et ALDH2*1/ALDH2*2 ;
    ii) ADH2*1/ADH2*2 et ALDH2*1/ALDH2*2 ; ou
    iii) ADH2*1/ADH2*2 et ALDH2*1/ALDH2*1.

**2.** 4-MP ou sel physiologiquement acceptable de celui-ci pour utilisation selon la revendication 1, où le symptôme d'intolérance à l'éthanol est choisi dans le groupe constitué par les suivants : bouffées de chaleur, augmentation du rythme cardiaque, palpitations, hypotension, nausées, étourdissements, céphalées, vomissements, diarrhées, perturbations gastriques, ataxie et confusion.

**3.** 4-MP ou sel physiologiquement acceptable de celui-ci pour utilisation selon la revendication 1, où la méthode comprend l'administration de 4-MP sous forme de base libre ou sous une forme saline physiologiquement acceptable.

**4.** 4-MP ou sel physiologiquement acceptable de celui-ci pour utilisation selon la revendication 1, où la méthode comprend l'administration de 4-MP ou d'un sel physiologiquement acceptable de celui-ci par voie orale avant que le sujet ne consomme d'éthanol, ou simultanément à la consommation d'éthanol du sujet, ou encore après que le sujet a consommé de l'éthanol.

**5.** 4-MP ou sel physiologiquement acceptable de celui-ci pour utilisation selon la revendication 4, où la méthode comprend l'administration de 4-MP ou d'un sel physiologiquement acceptable de celui-ci par voie orale entre environ une heure et environ 15 minutes avant que le sujet ne consomme d'éthanol.

**6.** 4-MP ou sel physiologiquement acceptable de celui-ci pour utilisation selon la revendication 1, où le pourcentage de réduction de la vitesse d'élimination d'éthanol du sujet n'est pas supérieur à environ 10 % par rapport à la vitesse d'élimination de l'éthanol d'un sujet auquel on n'a pas administré de 4-MP.

**7.** 4-MP ou sel physiologiquement acceptable de celui-ci pour utilisation selon la revendication 1, où la quantité de 4-MP ou du sel physiologiquement acceptable de celui-ci et efficace dans la réduction ou l'inhibition de l'activité oxydant l'éthanol de l'alcool déhydrogénase chez le sujet.

**8.** 4-MP ou sel physiologiquement acceptable de celui-ci pour utilisation selon la revendication 7, où le symptôme d'intolérance à l'éthanol est choisi dans le groupe constitué par les suivants : bouffées de chaleur, augmentation du rythme cardiaque, palpitations, hypotension, nausées, étourdissements, céphalées, vomissements, diarrhées, perturbations gastriques, ataxie et confusion.

**9.** Méthylpyrazole (4-MP) ou sel physiologiquement acceptable de celui-ci pour utilisation dans une méthode de réduction d'un risque chez un sujet pour une maladie ou à un trouble provoqués par la consommation d'éthanol, où la méthode comprend l'administration de 4-MP ou d'un sel physiologiquement acceptable de 4-MP à une teneur comprise entre 0,5 et 2,0 mg/kg de 4-MP ou la masse équivalente de 4-MP sous une forme saline physiologiquement acceptable, qui est efficace dans l'augmentation du catabolisme de l'acétaldéhyde chez le sujet, où l'acétaldéhyde est un produit de la consommation d'éthanol par le sujet et où l'augmentation du catabolisme de l'acétaldéhyde réduit un risque de maladie ou de trouble chez le sujet provoqué par une exposition à l'acétaldéhyde ;
et où le sujet présente le génotype suivant :

    i) ADH2*1/ADH2*1 et ALDH2*1/ALDH2*2 ;
    ii) ADH2*1/ADH2*2 et ALDH2*1/ALDH2*2 ; ou
    iii) ADH2*1/ADH2*2 et ALDH2*1/ALDH2*1.

**10.** 4-MP ou sel physiologiquement acceptable de celui-ci pour utilisation dans l'une ou l'autre des revendications 1 et 9, où le sujet est un humain.

**11.** 4-MP ou sel physiologiquement acceptable de celui-ci pour utilisation selon la revendication 1, où la maladie ou le trouble comprennent les cancers des voies aérodigestives supérieures, les cancers de l'appareil digestif ou le cancer du sein, ou où la maladie ou le trouble comprennent les suivants : maladie d'Alzheimer à déclenchement tardif, hypertension, infarctus du myocarde, maladie de Parkinson, sclérose latérale amyotrophique et ischémie cérébrale.

**12.** 4-MP ou sel physiologiquement acceptable de celui-ci pour utilisation selon la revendication 11, où le cancer des voies aérodigestives supérieures comprend le cancer de l'oesophage, de l'oropharynx, de l'hypopharynx, du larynx, de la tête ou du cou, ou où le cancer digestif comprend le cancer de l'estomac ou le cancer du côlon.

**13.** 4-MP ou sel physiologiquement acceptable de celui-ci pour utilisation selon l'une ou l'autre des revendications 1 et 9, où la méthode comprend l'administration d'une quantité active d'un sel de chlorhydrate de 4-MP.

**14.** 4-MP ou sel physiologiquement acceptable de celui-ci pour utilisation selon l'une ou l'autre des revendications 1 et 9, où la méthode comprend l'administration d'environ 1 milligramme de 4-MP par kilogramme du poids corporel du sujet.

**Fig. 1**

ADH                    ALDH

Ethanol        Acetaldehyde        Acetic acid

# Fig. 2

Fig. 3

# Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61185884 A
- US 2008021083 A
- WO 2005085392 A
- JP S57106620 B

### Non-patent literature cited in the description

- **BOSRON et al.** *Hepatology,* 1986, vol. 6, 502-510
- **MATSUO et al.** *Carcinogenesis,* 2006, vol. 27 (5), 1018-1023
- **TAMAKOSHI et al.** *Alcohol,* 2003, vol. 38, 407-410
- **YOSHIDA et al.** *Prog. Nucleic Acid Res. Mol. Biol.,* 1991, vol. 40, 255-287
- **CRABB et al.** *Proc. Nutr. Soc.,* 2004, vol. 63 (1), 49-63
- **CHEN et al.** *Am. J. Hum. Genet.,* 1999, vol. 65 (3), 795-807
- **VISAPÄÄ et al.** *Gut,* 2004, vol. 53, 871-876
- **YOKOYAMA et al.** *Jpn. J. Clin. Oncol.,* 2003, vol. 33 (3), 111-121
- **OHSAWA et al.** *J. Hum. Genet.,* 2003, vol. 48, 404-409
- **GOEDDE et al.** *Hum. Genet.,* 1992, vol. 88, 344-346
- **XIAO et al.** *J. Clin. Invest.,* 1995, vol. 96, 2180-2186
- **YIN, S. et al.** *Biochemical Genetics,* 1992, vol. 30 (3-4), 344-346
- **INOUE.** *Alcoholism: Clinical and Experimental Research,* 1984, vol. 8, 319-322
- **INOUE et al.** *Alcoholism: Clinical and Experimental Research,* 1984, vol. 8, 319-322
- **STOWELL.** *Clin. Chim. Acta.,* 1979, vol. 98, 201-5
- **MCCARVER-MAY et al.** *Journal of Analytical Toxicology,* 1997, vol. 21, 134-141
- **NAGY et al.** *Rapid Communications in Mass Spectrometry,* 2004, vol. 18, 2473-2478
- Longman Dictionary of Contemporary English. 1995
- **TRUETT et al.** *Biotechniques,* 2000, vol. 29 (1), 52-54